# EUROPEAN PATENT SPECIFICATION

(11) **EP 2 789 317 B1**
(45) Date of publication and mention of the grant of the patent: **13.12.2017**
(21) Application number: 13163345.5
(22) Date of filing: 11.04.2013
(51) Int. Cl.: A61F 5/445

(54) **Medical bag with a closing assembly**
Medizinischer Beutel mit einer Verschlussanordnung
Poche médicale avec ensemble de fermeture

(43) Date of publication of application: 15.10.2014
(73) Proprietor: OxMed International GmbH, 46446 Emmerich am Rhein (DE)
(72) Inventor: De Weert, Heleen, 7001 KJ Doetinchem (NL)
(74) Representative: Nederlandsch Octrooibureau

(56) References cited:
- EP-A1- 1 400 222
- WO-A2-2005/041828
- GB-A- 2 434 316
- US-A- 4 755 177
- US-A1- 2008 232 722
- US-B1- 6 231 553

## Description

### Field of the invention

The present invention relates to a medical device for use in collecting bodily waste material comprising a main pouch with an attachment area for attachment to a patient body interface. Exemplary implementations include an ostomy bag and a wound management bag.

### Prior art

In numerous medical applications bags or pouches are being used for various purposes, mostly for sealing or collecting purposes of all kinds of bodily fluids or material. Specific applications include wound management, where a usually larger wound area of a patient needs to be sealed off from the environment, and ostomy applications, where a collecting pouch is connected to a stoma of a patient.

In known wound management devices, a simple opening is provided in a wound management bag, in order to allow access to the wound or wounded area for treatment purposes. This opening is closed using e.g. a zipper type of sealing device. However, these are difficult to operate (especially by the patient) and provide insufficient sealing capacity, as a result of which wound fluids or odors can leave the bag. In effect, the patient is then immobilized.

An ostomy pouching system or bag is a medical device prosthetic that provides a means for the collection of waste from a surgically diverted biological system (colon, ileum, urinary) and the creation of a stoma. Pouching systems are most commonly associated with colostomies, ileostomies, and urostomies. Pouching systems usually consist of a collection pouch bag, known as a one-piece system or, in some instances involves a mounting plate, commonly called a wafer or a base plate, and a collection pouch that is attached mechanically or with an adhesive in an airtight seal, known as a two-piece system. Ostomy pouching systems collect waste that is output from a stoma. The pouching system allows the stoma to drain into a sealed collection pouch, while protecting the surrounding skin from contamination. The two-piece system requires regular replacement of the bag, which can be cumbersome for the patient.

British patent publication GB-A-2434316, discloses an ostomy drainage bag assembly for receiving bodily waste. The drainage bag assembly comprising an outer and inner bag secured to one side of a flange, the flange being provided with means defining an orifice to enable bodily waste to be received by the inner bag. At an opposite side of the flange means for securing the drainage bag assembly to the body of a patient are present. In an embodiment an opening may be formed in the outer bag along a seam, the opening being closed by a temporary closure mechanism, such as a clip and folding mechanism using, for example, a physical interlocking system such as a closure of the "Ziploc®" type. Document US 2008/0232722 A1 discloses a re-closable storage bag with a closure system comprising a first and second zipper which can be individually or simultaneously closed to seal contents within the bag.

### Summary of the invention

The present invention seeks to provide an improved solution to the above mentioned problems, notably in the area of sealing, both for fluids and odors.

According to the present invention, a medical device as defined in the preamble of claim 1 is provided, wherein the main pouch comprising a front side having a re-closable aperture therein, wherein the front side furthermore comprises a sealing assembly of a double line-type, and wherein the double line-type sealing assembly comprises two sealing assemblies, positioned parallel to each other. This is a simple and economic implementation of the double line-type sealing assembly, and provides for an improved sealing of the medical device, yet allows easy opening and closing thereof by the patient self or nursing staff. The double line-type sealing assembly extends over a major length of the front side of the main pouch, providing a good access to the inner side of the main pouch. The double line-type sealing assembly is positioned off-center on the front side in a further embodiment, allowing more convenient access for certain applications of the medical device.

The front side of the main pouch comprises pulling areas extending from the double line-type sealing assembly, in order to provide a convenient use for opening the main pouch. Each sealing assembly comprises a first sealing part attached to a first part of the front side of the main pouch and a second sealing part attached to a second part of the front side of the main pouch, the first sealing part and second sealing part having a congruent and point symmetric cross section. This implementation is simple to construct and manufacture, yet at reasonable material and manufacturing costs. The first sealing part and second sealing part are provided with a glue material in an even further, alternative embodiment.

The medical device is an ostomy bag in a further embodiment, and further comprises a secondary pouch positioned inside of the main pouch. The sealing assembly may be provided in a bottom part of the main pouch. The main pouch and secondary pouch are attachable to different parts of the patient body interface in a further embodiment. The secondary pouch may be removable from the interior of the main pouch during actual use of the medical device, via the re-closable aperture, increasing ease of use.

In a further group of embodiments, the medical device is a wound management bag, comprising a wound treatment device, the main pouch being attached to the wound treatment device for sealing off the wound treatment device. Also for this application the present invention medical device provides improvements over existing devices, especially in sealing efficiency and ease of opening and closing.

A further aspect of the present invention relates to a method for manufacturing a medical device according to any one of the embodiments described herein, comprising attaching the sealing assembly to the main pouch using a heat sealing technique. The method may further comprise manufacturing the main pouch by attaching a front side and back side of the main pouch using fusing or welding of the main pouch material.

### Short description of drawings

The present invention will be discussed in more detail below, using a number of exemplary embodiments, with reference to the attached drawings, in which
Fig. 1a shows a front view of a colostomy bag according to an embodiment of the present invention;
Fig. 1b shows a cross sectional view of the colostomy bag of Fig. 1b;
Fig. 1c shows a front view of the colostomy bag of Fig. 1a and b when opening;
Fig. 2a shows a front view of a wound management bag according to a further embodiment of the present invention;
Fig. 2b shows a front view of the wound management bag of Fig. 2a when opening; and
Fig. 3 shows a cross sectional view of an example of a sealing assembly as used in an embodiment of the present invention.

### Detailed description of exemplary embodiments

In numerous medical applications medical devices in the form of bags or pouches are being used for various purposes, mostly for sealing of an environment or for collecting purposes of all kinds of bodily waste material (faeces, urine, wound excretions). Specific applications include wound management, where a usually larger wound area of a patient needs to be sealed off from the environment, and ostomy applications, where a collecting pouch is connected to a stoma of a patient.

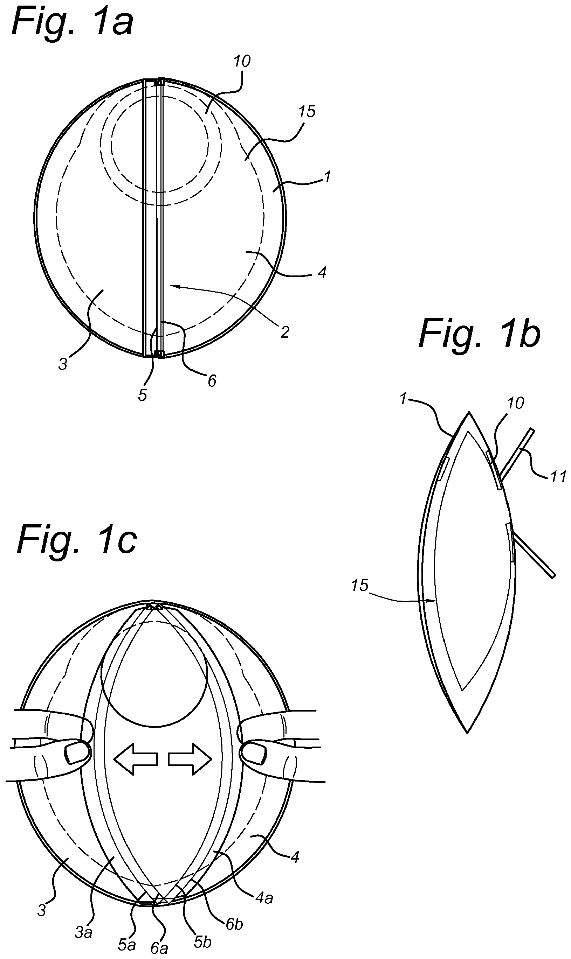

In a first embodiment of the present invention, an ostomy bag is provided, of which a front view and cross sectional view are shown in Fig. 1a and 1b. In Fig. 1c, a front view is shown of such an ostomy bag being opened.

An ostomy bag 1 forms a main pouch 1 of a medical device for collecting bodily waste material. The main pouch 1 is closed and sealed along an aperture using a sealing assembly 2. The aperture is formed between two parts 3, 4 of a front side of the main pouch 1, as is clearly shown in Fig. 1c.

Fig. 1a shows in dashed lines that the ostomy bag comprises a secondary pouch 15 or inner pouch, positioned inside the main pouch 1. Furthermore an area 10 is indicated (usually having a round shape as shown) which is an attachment area 10 for attaching the ostomy bag 1 to a patient body interface, such as an ostomy base plate 11. Fig. 1b shows the cross section of the ostomy bag 1 of Fig. 1a, attached to the ostomy base plate 11. The inner pouch 15 is in this embodiment attached to the opposite side of the ostomy base plate 11 during actual use. Alternatively, the secondary pouch 15 is attached to the main pouch 1, which in turn is attached to the ostomy base plate 11. In both cases, the secondary pouch 15 can be accessed from the main pouch 1 via an aperture having an interlocking plastic moulded closure forming the sealing assembly 2 (e.g. in the form of a minigrip, gripseal, zipper or ziplock closure).

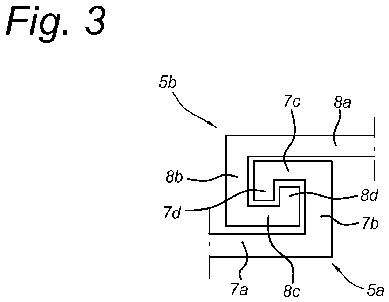

In other words, the sealing assembly is of a double line-type using a continuous line seal along its entire length by pressure acting between the sealing assembly parts. This is discussed in more detail below with reference to Fig. 3.

The sealing assembly 2 (and thus the aperture of the main pouch 1) can be positioned longitudinally or laterally on the north to south or east to west axis of the main pouch 1. In other embodiments, the double line-type sealing assembly 2 extends over a major length of the front side 3, 4 of the main pouch 1. The double line-type sealing assembly 2 is positioned off-center on the front side 3, 4 of the main pouch 1 in an even further embodiment. The sealing assembly 2 is of a double line-type each having two separate sealing assemblies 5, 6 positioned in parallel to each other, in a further embodiment.

As shown in the embodiment of Fig. 1c, the front side 3, 4 of the main pouch 1 comprises pulling areas 3a, 4a extending from the double line-type sealing assembly, in order to provide an easy grip for pulling open the main pouch 1.

In a specific embodiment, the sealing assembly 2 is provided in a bottom part of the main pouch 1, e.g. spanning the entire width of the main pouch 1. This would allow a patient to open and empty an ostomy bag while keeping the ostomy bag in place on the stoma.

As shown in more detail in the cross sectional view of Fig. 3, the profile of the sealing assembly 2 comprises two parts 5a, 5b, which are designed to interlock with each other along the length of the aperture of the main pouch 1, thereby providing a leak free closure. The sealing assembly 2 may comprise a double length of interlocking profiles 5a, 5b, 6a, 6b as shown in the embodiments of Fig. 1a-c. The double locking profile 5a, 5b, 6a, 6b will provide a more secure method of closure of the main pouch 1.

In an alternative arrangement, the medical device in the form of the ostomy bag comprises a single length of interlocking profiles 5a, 5b forming the sealing assembly 2, designed to interlock with each other along the length of the aperture of the main pouch 1. In other words, an ostomy bag is provided for use in collecting bodily waste material comprising a main pouch 1 with an attachment area 10 for attachment to a patient body interface 11, and a secondary pouch 15 positioned inside of the main pouch 1, the main pouch 1 comprising a front side 3, 4 having a re-closable aperture therein, wherein the front side 3, 4, furthermore comprises a sealing assembly 2 of a single line-type.

Fig. 3 shows in more detail a possible implementation of the sealing assembly 2 with the two interlocking profiles 5a, 5b; 6a, 6b. Similar as shown in the embodiment of Fig. 1c, the sealing assembly 2 comprises a first sealing part 5a; 6a attached to a first part 3 of the front side of the main pouch 1 and a second sealing part 5b; 6b attached to a second part 4 of the front side of the main pouch 1. The first sealing part 5a; 6a and second sealing part 5b; 6b have a congruent and point symmetric cross section as shown in the example of Fig. 3, thus providing a very good sealing and closing effect. The example implementation of Fig. 3 is arranged to have sealing parts 5a, 5b with at least three consecutive and perpendicular legs 7a-7d; 8a-8d of assembly. In other embodiment, the sealing parts 5a, 5b; 6a; 6b may be implemented as cross sectional parts or legs together extending over a bend of more than 180° to ensure proper gripping of both elements 5a, 5b; 6a, 6b. As further alternatives the first sealing part 5a, 6a and second sealing part 5b; 6b may use glue or another sticky material to provide the closing and sealing effect.

In all of the embodiments described above, the purpose of the zipper or grip seal product (sealing assembly 2) is to enable access to the inner pouch 15 in order to remove it when full. A replacement inner pouch or bag 15 may then be attached via the same opening in the main pouch 1. To open the aperture in the main pouch 1, sections of the film used to make the main pouch 1 (i.e. pulling areas 3a, 4a of the front side 3, 4 of the main pouch 1) close to the sealing assembly 2, are pulled apart revealing the inside of the main pouch 1. Further pulling exposes more of the inside of the main pouch 1 with improved access to the inner pouch 15. Closure of the main pouch 1 is achieved by bringing the two mating closure elements 5a, 5b; 6a, 6b together and performing a complete seal by pushing the elements 5a, 5b; 6a, 6b together along the entire length of the aperture.

In a further aspect in relation to the ostomy bag, a method is provided for manufacturing a medical device according to any one of the embodiments described herein, comprising attaching the sealing assembly 2 to the main pouch 1 using a heat sealing technique. The method may further comprise manufacturing the main pouch 1 by attaching a front side 3, 4 and a back side of the main pouch 1 using fusing or welding of the main pouch material. This step may even be executed after attachment of the sealing assembly 2 to the front side 3, 4. In other words, the front side 3, 4 (assembled using pouch panels of film with pre assembled sealing assemblies 2, i.e. heat sealed to the panels 3, 4), are brought together in conjunction with the a back side, and the two panels are fused or welded together to form a complete main pouch 1.

The ostomy bag disclosed and described above in multiple embodiments provides a number of benefits. The interlocking closure or sealing assembly 2 used in this invention is easy to open even for people with limited dexterity and is easy to close even for people with limited dexterity. It provides a wide aperture to access the inner pouch 15 without risk of dislodging the entire ostomy bag from the patients skin as opening requires the same but opposing forces (i.e. a pulling force is applied in opposite directions). When closed, the ostomy bag provides a leak and odour free seal, and thus improves the comfort and ease of the patient.

Especially for colostomy patients, this product is advantageous. When using existing ostomy bags, the patient cannot check regularly, and the output from the stoma can be very irregular (usually output occurs 2-3 times a day, but sometimes more frequent small quantities of output occur). If faeces is present in an ostomy bag, the patient will want to change directly. This increases the use of material, and is also very time consuming for the patient. Colostomy and ileostomy products are usually made of expensive materials, and the present invention embodiments thus can also provide an economical benefit.

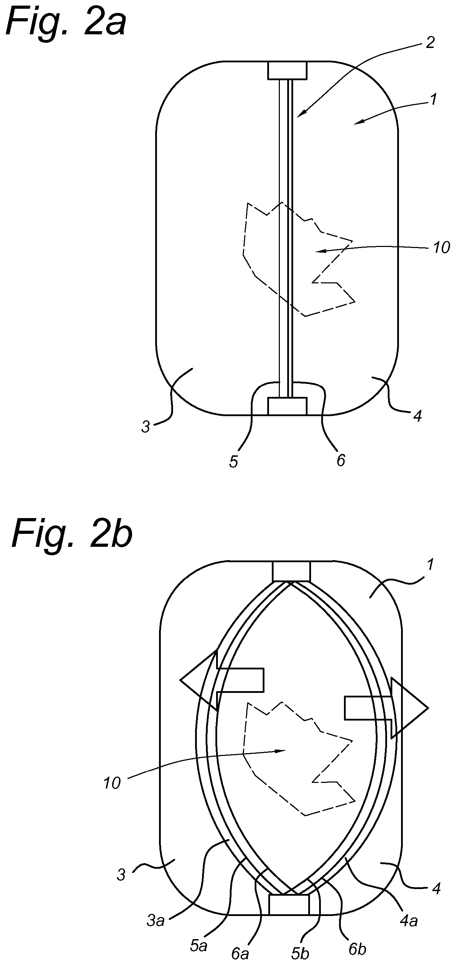

As a further application of the medical device of the present invention, the main bag 1 may form part of a wound management system in the form of a wound management bag 1. The aperture of the main pouch 1 then acts as a closure device for gaining access from a wound management pouch 1 to the surface area 10 of a wound (or to a patient body interface 11 such as a wound dressing or bandage), as shown in the front view of Fig. 2a. The wound 10 may be accessed via an interlocking plastic moulded closure formed by the sealing assembly 2, as shown in the front view of Fig. 2b.

As in the ostomy bag embodiments described with reference to Fig. 1, this closure can be positioned longitudinally or laterally on the north to south or east to west axis of the main pouch 1. The profile of the sealing assembly 2 again comprises two separate parts, as described above with reference to the ostomy bag embodiments and Figs. 1a-c and 3. The sealing assembly 2 comprises a double line-type of interlocking profiles 5, 6. In other words, two parallel locking profiles 5, 6, one independent from the other but both used together, providing more security from leakage than a single length of profile 5; 6 on its own. The double locking profile 5, 6 will provide a more secure method of closure. The zipper or grip seal interlocking elements 5, 6 of the sealing assembly 2 are permanently attached to the main pouch 1 using a heat sealing process method.

To open the sealing assembly 2, sections 3a, 4a of the pouch film 3, 4 close to the sealing assembly 2 itself to which the interlocking elements 5, 6 are attached, are pulled apart revealing the inside of the main pouch 1. Further pulling exposes more of the inside of the main pouch 1 and unrestricted access to the wound 10 for dressing and cleaning. Closure of the wound management bag 1 is achieved by bringing the two mating interlocking elements 5a, 5b; 6a, 6b together and performing a complete seal by pushing the two elements together along the entire axis of the sealing assembly 2.

The wound management bag 1 of this aspect of the present invention has a number of distinct advantages over prior art systems. The wound management bag 1 can be used many times without deterioration of performance, is easy to close even for people with limited dexterity and is easy to open for people with limited dexterity. It provides a wide aperture to access the wound 10 without risk of dislodging any associated product from the patients skin as opening requires the same but opposing forces in opposite directions. When closed, the wound management bag 1 provides a leak and odour free seal and provides a unique secure access and closure that other products on the market currently do not provide. Patients using such a wound management pouch 1 can again be mobile, without being hindered by possible leaks or odors.

The present invention embodiments have been described above with reference to a number of exemplary embodiments as shown in the drawings. Modifications and alternative implementations of some parts or elements are possible, and are included in the scope of protection as defined in the appended claims.

## Claims

1. Medical device for use in collecting bodily waste material comprising a main pouch (1) with an attachment area for attachment to a patient body interface (11),
the main pouch (1) comprising a front side (3, 4) having a re-closable aperture therein, wherein the front side (3, 4) furthermore comprises a sealing assembly (2) of a double line-type, **characterised in that** the double line-type sealing assembly (2) comprises two sealing assemblies (5, 6), positioned parallel to each other, wherein
each sealing assembly (5, 6) comprises a first sealing part (5a; 6a) attached to a first part (3) of the front side (3, 4) of the main pouch (1) and a second sealing part (5b; 6b) attached to a second part (4) of the front side (3, 4) of the main pouch (1), the first sealing part (5a; 6a) and second sealing part (5b; 6b) having a congruent and point symmetric cross section and being configured to interlock with each other.

2. Medical device according to claim 1, wherein the double line-type sealing assembly (2) extends over a major length of the front side (3, 4) of the main pouch (1).

3. Medical device according to claim 1 or 2, wherein the double line-type sealing assembly (2) is positioned off-center on the front side (3, 4).

4. Medical device according to any one of claims 1-3, wherein the front side (3, 4) of the main pouch (1) comprises pulling areas (3a, 4a) extending from the double line-type sealing assembly (2).

5. Medical device according to any one of claims 1-4, wherein the medical device is an ostomy bag, and further comprises a secondary pouch (15) positioned inside of the main pouch (1).

6. Medical device according to claim 5, wherein the sealing assembly (2) is provided in a bottom part of the main pouch (1).

7. Medical device according to claim 5 or 6, wherein the main pouch (1) and secondary pouch (15) are attachable to different parts of the patient body interface (11).

8. Medical device according to claim 5, 6, or 7, wherein the secondary pouch (15) is removable from the interior of the main pouch (1) during actual use of the medical device, via the re-closable aperture.

9. Medical device according to any one of claims 1-4, wherein the medical device is a wound management bag, comprising a wound treatment device (11),
the main pouch (1) being attached to the wound treatment device for sealing off the wound treatment device (11).

10. Method for manufacturing a medical device according to any one of claims 1-9, comprising attaching the sealing assembly (2) to the main pouch (1) using a heat sealing technique.

11. Method according to claim 10, further comprising manufacturing the main pouch (1) by attaching a front side (3, 4) and back side of the main pouch (1) using fusing or welding of the main pouch (1) material.

## Patentansprüche

1. Medizinische Vorrichtung für eine Verwendung beim Sammeln von Körperabfallmaterial, mit einem Hauptbeutel (1) mit einem Befestigungsbereich zum Befestigen an einer Patientenkörperschnittstelle (11),
wobei der Hauptbeutel (1) eine Vorderseite (3, 4) aufweist, die eine wiederverschließbare Öffnung in ihr hat, wobei die Vorderseite (3, 4) darüber hinaus eine Abdichtbaugruppe (2) einer Doppellinienart aufweist, **dadurch gekennzeichnet, dass** die Abdichtbaugruppe (2) der Doppellinienart zwei Abdichtbaugruppen (5, 6) aufweist, die parallel zueinander positioniert sind, wobei
jede Abdichtbaugruppe (5, 6) ein erstes Abdichtteil (5a; 6a), das an einem ersten Teil (3) der Vorderseite (3, 4) des Hauptbeutels (1) angebracht ist, und ein zweites Abdichtteil (5b; 6b) aufweist, das an einem zweiten Teil (4) der Vorderseite (3, 4) des Hauptbeutels (1) angebracht ist, wobei das erste Abdichtteil (5a; 6a) und das zweite Abdichtteil (5b; 6b) einen kongruenten und punktsymmetrischen Querschnitt haben und so aufgebaut sind, dass sie miteinander in Eingriff stehen.

2. Medizinische Vorrichtung gemäß Anspruch 1, wobei die Abdichtbaugruppe (2) der Doppellinienart sich über eine Hauptlänge der Vorderseite (3, 4) des Hauptbeutels (1) erstreckt.

3. Medizinische Vorrichtung gemäß Anspruch 1 oder 2, wobei die Abdichtbaugruppe (2) der Doppellinienart außerzentrisch an der Vorderseite (3, 4) positioniert ist.

4. Medizinische Vorrichtung gemäß einem der Ansprüche 1 bis 3, wobei die Vorderseite (3, 4) des Hauptbeutels (1) Zugbereiche (3a, 4a) aufweist, die sich von der Abdichtbaugruppe (2) der Doppellinienart erstrecken.

5. Medizinische Vorrichtung gemäß einem der Ansprüche 1 bis 4, wobei die medizinische Vorrichtung ein Stomabeutel ist und des Weiteren einen Sekundärbeutel (15) aufweist, der im Inneren des Hauptbeutels (1) positioniert ist.

6. Medizinische Vorrichtung gemäß Anspruch 5, wobei die Abdichtbaugruppe (2) in einem Bodenteil des Hauptbeutels (1) vorgesehen ist.

7. Medizinische Vorrichtung gemäß Anspruch 5 oder 6, wobei der Hauptbeutel (1) und der Sekundärbeutel (15) an verschiedenen Teilen der Patientenkörperschnittstelle (11) anbringbar sind.

8. Medizinische Vorrichtung gemäß Anspruch 5, 6 oder 7, wobei der Sekundärbeutel (15) aus dem Inneren des Hauptbeutels (1) während einer tatsächlichen Anwendung der medizinischen Vorrichtung über die wiederverschließbare Öffnung entfernbar ist.

9. Medizinische Vorrichtung gemäß einem der Ansprüche 1 bis 4, wobei die medizinische Vorrichtung ein Wundbehandlungsbeutel ist, der eine Wundbehandlungsvorrichtung (11) aufweist, wobei der Hauptbeutel (1) an der Wundbehandlungsvorrichtung anbringbar ist, um die Wundbehandlungsvorrichtung (11) zu versiegeln.

10. Verfahren zum Herstellen einer medizinischen Vorrichtung gemäß einem der Ansprüche 1 bis 9, mit dem Schritt Anbringen der Abdichtbaugruppe (2) an dem Hauptbeutel (1) unter Verwendung einer Wärmeabdichttechnik.

11. Verfahren gemäß Anspruch 10, das des Weiteren den folgenden Schritt aufweist: Herstellen des Hauptbeutels (1) durch Befestigen einer Vorderseite (3, 4) und einer Rückseite des Hauptbeutels (1) unter Verwendung eines Schmelzens oder Schweißens des Materials des Hauptbeutels (1).

## Revendications

1. Dispositif médical destiné à être utilisé pour collecter un matériau de déchet corporel, comprenant une poche principale (1) pourvue d'une zone de fixation pour être fixée à une interface (II) du corps d'un patient,
la poche principale (1) comprenant un côté avant (3, 4) muni d'une ouverture refermable,
dans lequel le côté avant (3, 4) comprend en outre un ensemble d'étanchéité (2) du type à double lignes, **caractérisé en ce que** l'ensemble d'étanchéité (2) du type à double lignes comprend deux ensembles d'étanchéité (5, 6) disposés parallèlement l'un à l'autre,
dans lequel chaque ensemble d'étanchéité (5, 6) comprend une première partie d'étanchéité (5a ; 6a) fixée à une première partie (3) du côté avant (3, 4) de la poche principale (1) et une deuxième partie d'étanchéité (5b ; 6b) fixée à une deuxième partie (4) du côté avant (3, 4) de la poche principale (1), la première partie d'étanchéité (5a ; 6a) et la seconde partie d'étanchéité (5b ; 6b) ayant une section transversale congruente et à symétrie ponctuelle et étant configurées pour s'emboîter l'une avec l'autre.

2. Dispositif médical selon la revendication 1, dans lequel l'ensemble d'étanchéité (2) du type à double lignes s'étend sur une longueur importante du côté avant (3, 4) de la poche principale (1).

3. Dispositif médical selon la revendication 1 ou 2, dans lequel l'ensemble d'étanchéité (2) du type à double lignes est décentré sur le côté avant (3, 4).

4. Dispositif médical selon l'une quelconque des revendications 1 à 3, dans lequel le côté avant (3, 4) de la poche principale (1) comprend des zones de traction (3a, 4a) s'étendant depuis l'ensemble d'étanchéité (2) du type à double lignes.

5. Dispositif médical selon l'une quelconque des revendications 1 à 4, dans lequel le dispositif médical est un sac de stomie, et comprend en outre une poche secondaire (15) positionnée à l'intérieur de la poche principale (1).

6. Dispositif médical selon la revendication 5, dans lequel l'ensemble d'étanchéité (2) est prévu dans une partie inférieure de la poche principale (1).

7. Dispositif médical selon la revendication 5 ou 6, dans lequel la poche principale (1) et la poche secondaire (15) peuvent être fixées à différentes parties de l'interface (11) du corps du patient.

8. Dispositif médical selon la revendication 5, 6 ou 7,
dans lequel la poche secondaire (15) peut être retirée de l'intérieur de la poche principale (1) en cours d'utilisation du dispositif médical, via l'ouverture refermable.

9. Dispositif médical selon l'une quelconque des revendications 1 à 4, dans lequel le dispositif médical est un sac de gestion de plaies, comprenant un dispositif de traitement de plaies (11), la poche principale (1) étant fixée au dispositif de traitement de plaies pour sceller le dispositif de traitement de plaies (11).

10. Procédé de fabrication d'un dispositif médical selon l'une quelconque des revendications 1 à 9, comprenant une fixation de l'ensemble d'étanchéité (2) à la poche principale (1) par une technique de scellement à chaud.

11. Procédé selon la revendication 10, comprenant en outre une fabrication de la poche principale (1) par une fixation du côté avant (3,4) et du côté arrière de la poche principale (1) par fusion ou soudage de la matière de la poche principale (1).
